# FASCICULE DE BREVET EUROPEEN

(11) **EP 0 597 025 B1**
(45) Date de publication et mention de la délivrance du brevet: **23.09.1998**
(21) Numéro de dépôt: 92917637.8
(22) Date de dépôt: 30.07.1992
(51) Int. Cl.: A61K 7/48, A61K 7/06, A61K 31/665, A61K 9/127, C07F 9/655

(54) **UTILISATION D'UN PHOSPHATE DE TOCOPHEROL, OU DE L'UN DE SES DERIVES, POUR LA PREPARATION DE COMPOSITIONS COSMETIQUES OU PHARMACEUTIQUES ET COMPOSITIONS AINSI OBTENUES**
VERWENDUNG VON TOCOPHERYL-PHOSPHAT SOWIE EINER IHREN DERIVATE, FÜR DIE HERSTELLUNG VON KOSMETISCHE ODER PHARMAZEUTISCHE ZUSAMMENSETZUNGEN
USE OF A TOCOPHEROL PHOSPHATE OR ONE OF ITS DERIVATIVES, FOR THE PREPARATION OF COSMETIC OR PHARMACEUTICAL COMPOSITIONS AND COMPOSITIONS SO OBTAINED

(30) Priorité: 01.08.1991 FR 9109825
(43) Date de publication de la demande: 18.05.1994
(73) Titulaire: LVMH RECHERCHE, 92752 Nanterre (FR)
(72) Inventeur: MEYBECK, Alain, F-92400 Courbevoie (FR); DUMAS, Marc, F-92700 Colombes (FR); BONTE. Frédéric, F-92400 Courbevoie (FR); MARECHAL, Christian, F-75003 Paris (FR)
(74) Mandataire: Portal, Gérard
(86) Numéro de dépôt international: FR9200748
(87) Numéro de publication internationale: WO9302661

(56) Documents cités:
- EP-A- 236 120
- EP-A- 376 162
- EP-A- 0 288 969
- EP-A- 0 332 478
- WO-A-89/03689
- WO-A-91/11189
- Chemical Abstracts, vol. 107, no. 10, 7 septembre 1987, (Columbus, Ohio, US), voir page 390, abrégé no. 83908d, & RO,A,89761 (INSTITUTUL ONCOLOGIC, BUCURESTI) 30 juillet 1986, voir abrégé
- Patent Abstracts of Japan, vol. 12, no. 391 (C-537)(3238), 18 octobre 1988, & JP,A,63139114 (SHISEIDO CO., LTD) 10 juin 1988, voir le document en entier

## Description

La présente invention concerne généralement l'utilisation d'un phosphate de tocophérol, ou de l'un de ses esters ou de ses sels, pour la préparation de compositions cosmétiques ou pharmaceutiques, notamment dermatologiques à activité anti-allergique, anti-inflammatoire ou destinés à la prévention ou au traitement des effets nocifs des radicaux libres, ainsi que les compositions cosmétiques ou pharmaceutiques, notamment dermatologiques à activité anti-allergique, anti-inflammatoire ou destinés à la prévention ou au traitement des effets nocifs des radicaux libres, l'incorporant.

On sait que la vitamine E a comme nom commun notamment l'alpha-tocophérol (voir Merck Index, 11^{ème} édition, Référence 9 832, page 1 437).

L'alpha-tocophérol se trouve à l'état naturel dans de nombreuses plantes, habituellement avec d'autres composés tels que le bêta-tocophérol, le gamma-tocophérol ou le delta-tocophérol.

On sait également que les tocophérols se présentent sous les deux formes dl ou d.

L'alpha-tocophérol est essentiellement utilisé pour lutter contre les déficiences en vitamine E, ou comme facteur nutritionnel, notamment pour lutter contre la dégénéréscence musculaire.

Il est également utilisé comme anti-oxydant, mais à des doses très spécifiques.

On a également décrit des esters d'α-tocophérol, et en particulier le succinate, le nicotinate ou l'acétate (Merck Index, 10^{ème} édition, Références 9 832, 9 833, page 1 437). La synthèse de l'acétate d'alpha-tocophérol est également décrite dans le document US-A-2 723 278, celle d'autres esters est décrite dans le document J. Amer. Chem. Soc. (1943) 65, 918-924.

Le phosphate de dl-alpha-tocophérol est également connu, (voir P. KARRER et ai., Helv. Chim. Acta, (1940) 23 1137-8) ainsi que son action sur le métabolisme musculaire (voir J. Biol. Chem. 1942, 146, pages 309-321). Un autre document décrit le rôle biologique d'anti-oxydant sur du tissu cérébral (Biol. anti-oxydants Trans., 1 st Conf., 1946, pages 61-62). Il a également été décrit une action anticoagulante par une action sur la polymérisation de la fibrine (Can. J. Biochem. and Physiol. 1959 37, pages 501-505). Une action anti-microbienne in vitro sur B. Subtilis et S. Aureus a également été décrite (Naturwissenchaften 1960, 47, page 17).

Les documents EP-A-0 236 120, EP-A-0 288 969, l'abrégé DERWENT WPIL data base No. 88-201765 (1988) en référence à la demande japonaise JP-63-139114 et US-A-5 053 222 décrivent des esters ascorbiques de monophosphates de tocophérol.

Le document Chem. Abs., 107, No.10, (1987), page 390, abrégé N°83908d, décrit un phosphate de tocophérol usuel dans une composition pharmaceutique.

Il a maintenant été découvert, de manière tout à fait surprenante et inattendue, que les phosphates de tocophérol autres que le phosphate d'alpha-tocophénol, notamment sous leur forme dl ou d, ou de l'un de leurs esters, ou de leurs sels possèdent une activité anti-allergique, anti-inflammatoire, et antiradicalaire, ce qui permet de les utiliser avantageusement pour la préparation de compositions cosmétiques ou pharmaceutiques, notamment dermatologiques, à potentiel allergisant ou irritant réduit, ou destinées à la prévention ou au traitement des manifestations allergiques, ou anti-inflammatoires, ou encore à la prévention ou au traitement des effets nocifs des radicaux libres.

Ainsi, la présente invention a pour but de résoudre le nouveau problème technique consistant en la fourniture d'une substance active présentant une bonne activité anti-allergique, notamment pour la prévention ou le traitement de l'allergie cutanée ou de l'asthme bronchique, ou anti-inflammatoire ou encore une activité préventive ou curative des effets nocifs des radicaux libres, en particulier par voie topique ou générale, en constituant ainsi un ingrédient actif précieux pour la préparation de compositions cosmétiques, ou pharmaceutiques, notamment dermatologiques.

La présente invention résout ce nouveau problème technique de manière satisfaisante, selon une solution particulièrement simple, utilisable à l'échelle industrielle.

Ainsi, selon un premier aspect, la présente invention couvre l'utilisation d'un phosphate de tocophérol autre que l'alpha-tocophérol, notamment sous sa forme dl ou d, ou de l'un de ses esters, de formule générale (I) suivante : 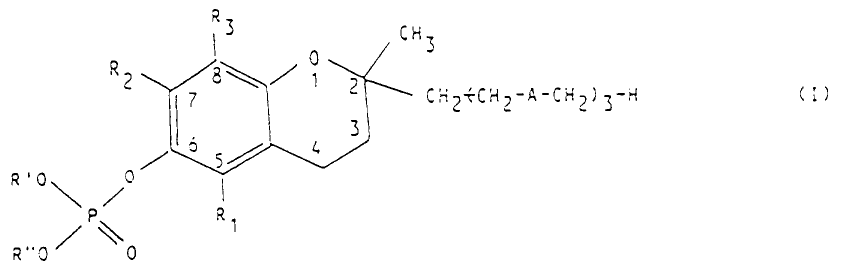 dans laquelle :
R' représente un atome d'hydrogène, un radical alkyle ayant de 1 à 4 atomes de carbone, tel qu'en particulier le radical méthyle ou éthyle, ou R'O représente un radical tocophéryle ;
R'' représente un atome d'hydrogène, un radical alkyle ayant de 1 à 4 atomes de carbone, tel qu'en particulier le radical méthyle ou éthyle, ou R''O représente une chaîne oxyéthylénée, de formule 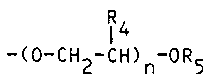 dans laquelle R₄ et R₅ représentent indépendamment un atome d'hydrogène ou un radical méthyle, et n représente un nombre entier supérieur ou égal à 1 ;
R₁, R₂ et R₃ représentent indépendamment un atome d'hydrogène ou un radical méthyle, étant entendu que R₁, R₂ et R₃ ne peuvent pas représenter simultanément un radical méthyle.
A représente les groupes : 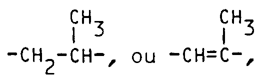 ou le dzéta 1-tocophérol ou 5,7,8-triméthyltocotriène-3',7',11'-ol, ou de l'un de ses sels, cosmétiquement ou pharmaceutiquement acceptables, pour la préparation d'une composition cosmétique ou pharmaceutique, notamment dermatologique, à potentiel allergisant ou irritant réduit, ou destiné à la prévention ou au traitement des manifestations allergiques, telles que l'allergie cutanée ou l'asthme bronchique, ou inflammatoire, ou à la prévention ou au traitement des effets nocifs des radicaux libres.

Dans la formule précédente, le phosphate de tocophérol précité préféré est le phosphate de tocol, de bêta-tocophérol ou 5,8-diméthyltocol ; gamma-tocophérol ou 7,8-diméthyltocol ; dzéta 1,2-tocophérol ou 5,7-diméthyltocol ; delta-tocophérol ou 8-méthyl-tocol ; êta-tocophérol ou 7-méthyltocol ; le tocotriénol, dzéta 1-tocophérol ou 5,7,8-triméthyltocotriène-3',7',11'-ol ; epsilon-tocophérol ou 5,8-diméthyltocotriène-3',7',11'-ol ; ou gamma-tocotriénol ou delta-tocotriénol.

Suivant une variante de l'utilisation selon la présente invention, on utilise un mélange de phosphates de tocophérol(s), dont l'un au moins est autre que le phosphate d'alpha-tocophérol, ou de leurs esters ou de leurs sels, obtenu notamment par phosphatation d'un mélange de tocophérols extrait d'un plante, telle que le soja.

La fabrication des phosphates de tocophérol peut être réalisée à partir du procédé bien connu de fabrication du phosphate d'alpha-tocophérol. Un procédé de préparation du phosphate d'alpha-tocophérol ainsi que de ses sels est décrit dans le document JP-A-37-1737 de Tomoda Pharm. Manuf. Co. Limited auquel l'homme de l'art pourra se reporter.

Ainsi, les produits utilisés conformément à la présente invention seront des phosphates de tocophérol, autres que le phosphate d'alpha-tocophérol, ou leurs esters, ces produits pouvant se présenter sous forme de sels cosmétiquement ou pharmaceutiquement, notamment dermatologiques, acceptables, tels que par exemple des sels de métaux alcalins, notamment de sodium (sel monosodique ou disodique), ou alcalino-terreux, notamment de magnésium, ou encore des sels d'ammonium ou d'amines primaires, secondaires ou tertiaires tels qu'en particulier la diéthylamine, la diéthanolamine, la triéthylamine ou la triéthanolamine.

Dans la formule (I), les radicaux alkyles peuvent être à chaîne droite ou ramifiée.

Un radical alkyle ayant de 1 à 4 atomes de carbone est par exemple méthyle, éthyle, propyle, isopropyle, butyle, de préférence méthyle ou éthyle.

Par radical tocophéryle, on entend désigner le radical de formule (II) suivante : 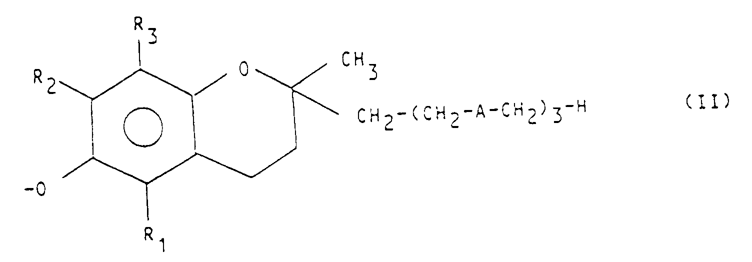 dans laquelle R₁, R₂ et R₃ représentent indépendemment un atome d'hydrogène, un radical méthyle, et A représente les groupes : 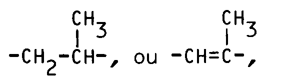 lorsque R''O représente une chaîne oxyéthylénée, n sera généralement supérieur ou égal à 1, par exemple compris entre 2 et 50, de préférence entre 2 et 25 et en particulier égal à 2 ou 5.

Suivant un autre mode de réalisation avantageux, conforme à l'invention, on utilise un phosphate de tocophérol, tel que précédemment défini, de préférence à l'état de sel, sous forme de petites vésicules de type liposome obtenues par dispersion dudit composé ou dudit sel dans l'eau ou dans un milieu aqueux, tel qu'une solution tampon, notamment au moyen d'une agitation mécanique suivie d'une homogénéisation, par exemple à l'aide d'ultra-sons ou d'un homogénéiseur sous pression.

De préférence, on règle la taille de ces vésicules à une valeur comprise environ entre 6.10⁻² µm et 2 µm, en modifiant les paramètres de l'homogénéisation tels que l'énergie et la durée.

Suivant une variante avantageuse du précédent mode de réalisation, le milieu aqueux précité contient un agent biologiquement actif, ledit agent étant encapsulé au moins en partie après dispersion dans les vésicules précitées.

De préférence, l'agent actif précité est une substance anti-allergique, telle qu'un extrait de Scutellaria, comme par exemple un extrait de racine de Scutellaria Baicalensis Georgi décrit dans le document FR-A-2 628 317, ou une substance anti-inflammatoire.

Selon un mode de réalisation avantageux de l'utilisation conforme à l'invention, la concentration en poids du phosphate de tocophérol précité, ou de l'un de ses sels, ou de leurs mélanges est comprise entre 0,001 et 10 %, de préférence entre 0,01 % et 1 %, et de préférence encore entre 0,05 % et 0,5 %, par rapport au poids total de la composition.

Selon un mode de réalisation actuellement préféré, le phosphate de tocophérol précitée est un phosphate de delta-tocophérol. Les sels préférés sont les sels monosodiques et le sel disodique.

Les composés utilisés conformément à l'invention sont généralement disponibles dans le commerce et peuvent être notamment préparés en suivant des processus décrits dans la littérature, par exemple dans : Chem. Pharm. Bull, (1971), 19, (4), pages 687 à 695 ; Khim.-Pharm. Zh (1983), 17, (7), pages 840 à 844 ; Khim.-Pharm. Zh (1985), 19, (1), pages 75 à 77 ou encore dans les brevets US-2 457 932 ou JP-54-54 978.

La nomenclature des tocophérols et des composés dérivés a été décrite dans Européenne J. Biochem., (1982), 123, 473-475. La préparation du delta-tocophérol a été décrite dans J. Am. Chem. Soc. (1947), 69, pages 869-874. De même, la préparation des alpha, bêta et gamma-tocophérol naturel et les certains esters d'intérêt physiologique a été décrite dans J. Am. Chem. Soc., (1943), 65, pages 918-924. Le document GB-A-900 085 décrit encore un procédé de fabrication de delta-tocophérol. La synthèse de dzeta-1-tocophérol, d'epsilon-tocophérol et des tocotriénols est décrite dans le document Helvetica Chimica Acta (1963), 46, pages 2517-2526. La synthèse d'êta-tocophérol ou 7-méthyl-tocol est décrite dans le document Nature (1956), 177, pages 86-87. D'autres données sur les tocophérols se trouvent dans le Merck Index bien connu à l'homme de l'art.

Selon un deuxième aspect, la présente invention couvre une composition cosmétique ou pharmaceutique, notamment dermatologique, à potentiel allergisant ou irritant réduit, ou destinée notamment à la prévention ou au traitement des manifestations allergiques telles que l'allergie cutanée ou l'asthme bronchique, ou inflammatoires, ou à la prévention ou au traitement des effets nocifs des radicaux libres, caractérisée en ce qu'elle comprend une quantité cosmétiquement ou pharmaceutiquement efficace d'un phosphate de tocophérol, autre que le phosphate d'alpha-tocophérol, notamment sous sa forme dl ou d, ou de l'un de ses esters, de formule générale (I) suivante : 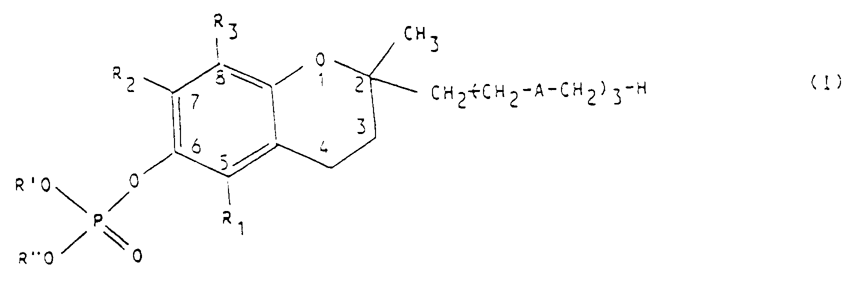 dans laquelle :
R' représente un atome d'hydrogène, un radical alkyle ayant de 1 à 4 atomes de carbone, tel qu'en particulier le radical méthyle ou éthyle, ou R'O représente un radical tocophéryle :
R'' représente un atome d'hydrogène, un radical alkyle ayant de 1 à 4 atomes de carbone, tel qu'en particulier le radical méthyle ou éthyle, ou R''O représente une chaîne oxyéthylénée, de formule 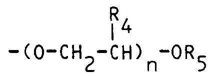 dans laquelle R₄ et R₅ représentent indépendamment un atome d'hydrogène ou un radical méthyle, et n représente un nombre entier supérieur ou égal à 1 ;
R₁, R₂ et R₃ représentent indépendamment un atome d'hydrogène ou un radical méthyle, étant entendu que R₁, R₂ et R₃ ne peuvent pas représenter simultanément un radical méthyle,
A représente les groupes : 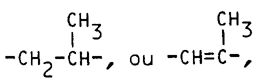 ou le dzéta 1-tocophérol ou 5,7,8-triméthyltocotriène-3',7',11'-ol, ou de l'un de ses sels, cosmétiquement ou pharmaceutiquement acceptables.

Selon une variante de réalisation, les compositions de l'invention comprennent à titre d'ingrédient actif un mélange de phosphates de tocophérol(s), dont l'un au moins est autre que le phosphate d'alpha-tocophérol, ou leurs esters, ou leurs sels, obtenus notamment par phosphatation d'un mélange de tocophérols extrait d'une plante, telle que le soja.

Selon un mode de réalisation avantageux, la composition cosmétique ou dermatologique comprend à titre d'ingrédient actif une quantité cosmétiquement ou pharmaceutiquement efficace d'un phosphate de tocophérol tel que précédemment défini, de préférence à l'état de sel, sous forme de petites vésicules de type liposome obtenues par dispersion dudit composé ou dudit sel dans l'eau ou dans un milieu aqueux tel qu'une solution tampon, notamment au moyen d'une agitation mécanique suivie d'une homogénéisation, par exemple à l'aide d'ultra-sons ou d'un homogénéiseur sous pression.

De préférence, on règle la taille de ces vésicules à une valeur comprise environ entre 6.10⁻² µm et 2 µm, en modifiant les paramètres de l'homogénéisation tels que l'énergie et la durée.

Suivant une variante avantageuse du précédent mode de réalisation, le milieu aqueux précité contient un agent biologiquement actif, ledit agent étant encapsulé au moins en partie après dispersion dans les vésicules précitées.

De préférence, l'agent actif précité est une substance anti-allergique, telle qu'un extrait de Scutellaria, comme par exemple un extrait de racine de Scutellaria Baicalensis Georgi décrit dans le document FR-A-2 628 317, ou une substance anti-inflammatoire.

Selon un autre mode de réalisation avantageux, lesdites compositions cosmétiques ou dermatologiques sont préparées en vue de présenter un potentiel allergisant ou irritant réduit, ou d'être destinées à la prévention et au traitement des manifestations allergiques, telles que l'allergie cutanée ou l'asthme bronchique, ou inflammatoires, ou à la prévention ou au traitement des effets nocifs des radicaux libres.

La concentration en ingrédients actifs, dans ces compositions cosmétiques ou dermatologiques est telle que décrite précédemment pour l'utilisation.

Les compositions selon l'invention peuvent être formulées selon toute forme acceptable pour leur emploi en cosmétologie, dermatologie ou pharmacie. Il peut en particulier s'agir de crème préventive et curative des allergies cutanées, de crème anti-allergique calmante, d'huile calmante anti-allergique, de lotion anti-allergique préventive ou curative, de lotion alcoolique après-rasage pour calmer les irritations de la peau, de crème hypo-allergénique, de solution colloïdale anti-asthmatique, ou encore de solution destinée à combattre les effets toxiques des radicaux super-oxydes qui se forment lors de la mise en oeuvre de techniques de réanimation avec l'oxygène.

Les compositions selon l'invention peuvent être également formulées sous forme de compositions de maquillage telles que fond de teint, rouge à lèvres, mascara, poudre teintée.

Selon un troisième aspect, la présente invention couvre un procédé pour diminuer le potentiel allergisant ou irritant d'une composition pharmaceutique, dermatologique ou cosmétique caractérisé en ce qu'il consiste à incorporer à ladite composition une quantité cosmétiquement ou pharmaceutiquement efficace d'au moins un phosphate de tocophérol ou d'au moins l'un de ses sels tels que précédemment définis.

Selon un mode de réalisation actuellement préféré, le composé de formule (I) précité est le phosphate de tocol, de bêta-tocophérol ou 5,8-diméthyltocol ; gamma-tocophérol ou 7,8-diméthyltocol ; dzéta 1,2-tocophérol ou 5,7-diméthyltocol ; delta-tocophérol ou 8-méthyltocol ; êta-tocophérol ou 7-méthyltocol ; tocotriénol, dzéta 1-tocophérol ou 5,7,8-triméthyltocotriène-3',7',11'-ol ; epsilon-tocophérol ou 5,8-diméthyltocotriène-3',7',-11'-ol ; ou gamma-tocotriénol ou delta-tocotriénol. Les sels préférés sont les sels monosodiques et le sel disodique.

Avantageusement, la concentration en phosphate de tocophérol est telle que décrite précédemment pour l'utilisation.

Selon une variante de réalisation de ce procédé, on incorpore un mélange de phosphates de tocophérol, dont l'un au moins est autre que le phosphate d'alpha-tocophérol, ou de l'un de leurs sels ou esters, obtenus notamment par phosphatation d'un mélange de tocophérols extrait d'une plante telle que le soja.

Selon un quatrième aspect, la présente invention concerne encore un procédé de fabrication d'une composition cosmétique ou dermatologique, à potentiel allergisant ou irritant réduit, ou destinée en particulier à la prévention ou au traitement des manifestations allergiques, telles que l'allergie cutanée, ou inflammatoires, ou à la prévention ou au traitement des effets nocifs des radicaux libres, caractérisé en ce qu'il comprend l'incorporation d'au moins un phosphate de tocophérol ou le dzéta 1-tocophérol ou 5,7,8-triméthyltocotriène-3',7',11'-ol de l'un de ses sels tels que précédemment définis dans un excipient, véhicule ou support cosmétiquement ou dermatologiquement acceptable.

Selon un cinquième aspect, la présente invention couvre encore un procédé de fabrication d'une composition pharmaceutique à potentiel allergisant ou irritant réduit, ou destinée à la prévention ou au traitement des manifestations allergiques telles que l'asthme bronchique, ou inflammatoires, ou à la prévention ou au traitement des effets nocifs des radicaux libres, caractérisé en ce qu'il comprend l'incorporation d'au moins un phosphate de tocophérol de formule (I) ou le dzéta 1-tocophérol ou 5,7,8-triméthylytocotriène-3',7',11'-ol, ou de l'un de ses sels tels que précédemment définis dans un excipient, véhicule ou support pharmaceutiquement acceptable.

L'incorporation du phosphate de tocophérol ou de l'un de ses sels dans ladite composition cosmétique, dermatologique ou pharmaceutique peut être effectuée selon différentes manières accessibles à l'homme de l'art, selon le type de formule désirée.

Suivant un mode avantageux de mise en oeuvre desdits procédés de fabrication, lorsque la composition comprend une phase aqueuse, le phosphate de tocophérol précité est dispersé, de préférence à l'état de sel tel que déjà défini, préalablement dans de l'eau ou dans ladite phase aqueuse pour former de petites vésicules, et la dispersion ainsi obtenue est ensuite mélangée aux autres constituants éventuels de la composition.

Selon un sixième aspect, la présente invention couvre un procédé de prévention ou de traitement des manifestations allergiques, telles que l'allergie cutanée ou l'asthme bronchique, ou inflammatoires, ou de prévention ou de traitement des effets nocifs des radicaux libres, caractérisé en ce qu'il comprend l'administration par voie générale, ou par voie topique, sur les régions du corps à traiter, d'une quantité cosmétiquement ou pharmaceutiquement efficace d'au moins un phosphate de tocophérol de formule (I) ou le dzéta 1-tocophérol ou 5,7,8-triméthyltocotriène-3',7',11'-ol, l'un de ses sels tels que précédemment définis, incorporés dans un excipient, véhicule ou support cosmétiquement, dermatologiquement ou pharmaceutiquement acceptable.

Selon un septième aspect, la présente invention couvre encore à titre de produits nouveaux le monophosphate ou diphosphate de tocol, d'epsilon-tocophérol, de dzéta 1 ou dzéta 2-tocophérol, d'êta-tocophérol, et le diphosphate de bêta, gamma ou delta-tocophérol, ainsi que leurs sels et esters.

L'invention sera maintenant illustrée en détail à l'aide de plusieurs exemples de réalisation donnés simplement à titre d'illustration et qui ne sauraient en aucune façon limiter la portée de l'invention.

Dans les exemples, les pourcentages sont donnés en poids sauf indication contraire.

### Exemple 1

### a) Préparation du sel disodique de phosphate de delta-tocophérol

Le schéma réactionnel est le suivant : 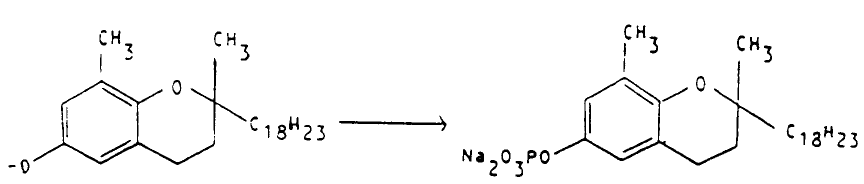 1) POCl₃, Et₃N, -20°C, 4 h. 2) H₂O, reflux, 3 h. 3) NaOH/MeOH

Dans un ballon de 500 ml muni d'une agitation magnétique, d'un thermomètre d'une ampoule de coulée et d'un ballon d'argon, on place 8,53 g (55,7 mmol) d'oxychlorure de phosphore dans 80 ml d'éther isopropylique préalablement distillé. On ajoute ensuite une solution de 20 g (49,6 mmol) de delta-tocophérol et de 9,04 g (89,3 mmol) de triéthylamine dans 200 ml d'éther isopropylique, à -20°C (l'addition dure environ 2 h). On laisse encore agiter pendant 2 h à -20°C puis on ajoute 20 ml d'eau en laissant la température remonter à la température ambiante. On chauffe ensuite à reflux pendant 3 h puis on refroidit. On soutire la phase aqueuse, évapore le milieu réactionnel au tiers de son volume, et on ajoute sous agitation une solution de 50 ml de soude méthanolique 10 %. On évapore le solvant et on lave la pâte jaunâtre obtenue avec 20 ml de méthanol refroidi à -20°C. La pâte est ensuite séchée à la pompe à palette à 60° pendant 12 h. On obtient ainsi 20,3 g (78 %) de sel disodique de delta-tocophérol.
Caractéristiques de t'échantillon ainsi obtenu :
RMN ³¹-P (CDCl₃)
5,9 ppm (monophosphate), -10,67 ppm (pyrophosphate)
Taux de sodium
% Na

| | |
|---|---|
| calculé | 8,73 % |
| trouvé | 5,86 %. |

### b) Préparation d'une solution de phosphate disodique de delta-tocophérol

On pèse 0,8 g de poudre de phosphate disodique de delta-tocophérol obtenu selon l'étape a) ci-dessus.

On verse cette poudre dans 96,2 g d'eau bidistillée, sous agitation que l'on poursuit pendant environ 2 heures.

On obtient ainsi la solution de phosphate disodique de delta-tocophérol.

### c) Préparation d'une suspension de phosphate acide de delta-tocophérol

On abaisse le pH de la solution obtenue à l'étape b) jusqu'à 7 sous agitation avec ajout d'environ 3 ml de HCl, 0,5 N, puis on ajuste le pH à 6,0 sous agitation avec HCl 0,1 N. A ce pH, le phosphate-tocophérol est alors sous forme acide.

On effectue ensuite une homogénéisation par ultrasons pendant 10 min à 150 W jusqu'à obtention d'une suspension limpide, donnant lieu à l'obtention de vésicules de type liposome de phosphate de tocophérol acide.

Dans le cas de volumes plus importants, on peut avantageusement utiliser un homogénéiseur sous pression, par exemple de type Manton-Gaulin® à la pression de 500 bars environ.

On peut déterminer la dimension des vésicules de phosphate acide de delta-tocophérol ainsi obtenue par exemple au moyen d'un Autosizer 2C de la société MALVERN. Dans cet exemple, la taille moyenne mesurée est de l'ordre de 130 nm.

On observera également que l'on peut réaliser diverses dilutions en modifiant la quantité de composés ajoutés au départ ou en modifiant le volume de la solution de dispersion, ce qui constitue un procédé aisé de préparation de diverses concentrations en principe actif.

Dans l'exemple décrit, on a obtenu environ 100 g de suspension contenant environ 0,8 % de phosphate acide de delta-tocophérol sous forme de vésicules de type liposome, de tailles sensiblement homogènes.

### d) Préparation d'une composition gélifiée de phosphate de delta-tocophérol

La suspension homogénéisée obtenue précédemment à l'étape c) peut être gélifiée par mélange avec un gel, tel qu'un gel de polymère vinylique, en particulier commercialisé sous la dénomination commerciale Carbopol® 940.

D'une façon connue en soi, on peut préparer ce gel par exemple en dispersant 1 g de Carbopol® 940 dans 99 g d'eau en présence d'un conservateur, puis, après gonflement, en neutralisant à pH 7,5, avec par exemple de la triéthanolamine.

Aux 100 g de suspension homogénéisée obtenue précédemment, on ajoute 100 g de ce gel, pour obtenir une composition gélifiée, dont la concentration en phosphate de delta-tocophérol est d'environ 0,4 %.

Des compositions gélifiées de concentrations variées en phosphate de delta-tocophérol peuvent être obtenues en suivant le procédé indiqué ci-dessus.

### Exemple 2

### Mise en évidence de l'inhibition de la phospholipase A₂ (ou PLA₂)

On mesure l'inhibition de la phospholipase A₂ qui intervient dans la production des médiateurs de l'allergie et de l'inflammation, notamment dans la réaction inflammatoire des allergies cutanées, selon le protocole décrit par H. W. Tchang, I. Kudo, M. Tomita et K. Inoue dans J. Biol. Chem., 1987, 102, 147-154.

On isole la phospholipase A₂ de la cavité péritonéale de rat. La PLA₂ hydrolyse la liaison ester en position 2 du glycérol d'une phosphatidyléthanolamine, cette position étant occupée par un reste d'acide gras marqué au carbone 14. L'acide gras radioactif libéré sera extrait puis dosé en scintillation liquide. En présence de l'inhibiteur de la PLA₂, cette hydrolyse sera plus faible et l'on dosera donc moins d'acide gras libéré.

En pratique, on met la PLA₂ et le produit à tester dans un tampon Tris-HCl 0,1 M, pH = 9, contenant 4 mmol de Ca⁺⁺ et on incube 20 min à 37°C sous agitation. Après ce temps de contact, on ajoute la phosphatidyléthanolamine marquée et on incube de nouveau 20 min à 37°C sous agitation pour permettre l'hydrolyse de ce substrat. On extrait alors, par du n-heptane,
l'acide gras marqué libéré au cours de l'hydrolyse, celui-ci étant alors dosé en scintillation liquide. On détermine alors la concentration d'inhibition 50 (CI₅₀), c'est-à-dire la concentration de produit à tester pour laquelle la quantité d'acide gras marqué libéré est la moitié de celle que l'on obtient en absence de produit (témoin).

Ainsi, avec le sel disodique de phosphate de delta-tocophérol préparé à l'étape b) de l'exemple 1, on obtient une CI₅₀ de 10 µg/ml. Cette concentration est faible et démontre une très bonne activité inhibitrice du sel disodique de phosphate de delta-tocophérol.

### Exemples de formules pharmaceutiques ou cosmétiques contenant du phosphate de vitamine E

### Exemple 3

### Crème préventive et curative des allergies cutanées.

### Composition :

| | | |
|---|---|---|
| A | Cera bellina | 5,00 g |
| | Silicone 200 | 1,50 g |
| | Squalane | 5,00 g |
| | Myglyol 812 | 5,00 g |
| | Nylon 12 SP 500 | 3,00 g |
| | BHT | 0,05 g |
| B | eau déminéralisée | 49,56 g |
| | EDTA | 0,10 g |
| | Propylène glycol | 4,00 g |
| | Carbopol® 1342 | 0,45 g |
| | Triéthanolamine | 0,54 g |
| | Dispersion de phosphate de delta-tocophérol à 0,4 %, pH 6,6 | 25,00 g |
| C | Germaben II® | 0,80 g |

Mode opératoire : On chauffe le mélange A sous agitation, afin d'obtenir un mélange homogène. Pour préparer le mélange B, on disperse le Carbopol® 1342 dans la solution aqueuse contenant l'EDTA et le propylène glycol dans 49,56 g d'eau distillée, et on neutralise avec la triéthanolamine. On ajoute ensuite la dispersion à 0,4 % (non gélifiée) de phosphate de delta-tocophérol obtrenue selon l'exemple 1.

On porte ensuite le mélange B à 75°C et on le maintient à cette température sous agitation pendant que l'on y ajoute le mélange A. On laisse refroidir à 45°C, puis on ajoute le Germaben II®, et on laisse refroidir encore, sous agitation, jusqu'à température ambiante.

On obtient ainsi une crème.

### Exemple 4

### Crème anti-allergique calmante

### Composition :

| | | |
|---|---|---|
| A | Lécithine de soja | 2,00 g |
| | Huile végétale | 8,50 g |
| B | Eau déminéralisée | 58,85 g |
| | EDTA | 0,10 g |
| | Glycérine | 4,00 g |
| | Carbopol® 940 | 0,35 g |
| | Triéthanolamine | 0,40 g |
| | Germaben II® | 0,80 g |
| C | Dispersion de phosphate delta-tocophérol à 0,4%, pH 6,6 | 25,00 g |

Mode opératoire : On chauffe sous agitation l'huile végétale et la lécithine jusqu'à dissolution complète, et on laisse refroidir à température ambiante. Le mélange B est obtenuen dispersant le Carbopol® 940 dans le mélange eau+EDTA+glycérine. On neutralise le tout avec la triéthanolamine, puis on ajoute le Germaben II®.

On verse ensuite sous agitation le mélange A sur le mélange B. On homogénéise, puis on ajoute la dispersion obtenue comme à l'exemple 1. On homogénéise encore et on obtient ainsi une crème utilisable matin et soir pour calmer les réactions allergiques cutanées en applications locales.

### Exemple 5

### Huile calmante anti-allergique

On dissout 0,1 g de poudre de phosphate de gamma-tocophérol disodique préparé selon une procédure similaire à celle de l'exemple 1, dans 99,9 g de trioctyle citrate sous agitation magnétique à 70°C pendant 8 h.

La solution huileuse ainsi obtenue peut être utilisée en applications locales, comme la crème de l'exemple 4.

### Exemple 6

### Lotion alcoolique après-rasage

### Composition :

| | |
|---|---|
| Phosphate de tocol disodique | 0,2 g |
| Ethanol | 40 g |
| Propylène glycol | 0,5 g |
| Pantothénol | 0,1 g |
| Excipient aqueux | |
| parfumé q.s.p. | 100 g |

Préparation : On dissout séparément le phosphate de tocol disodique, préparé selon une procédure similaire à celle de l'exemple 1, dans l'alcool absolu d'une part, et les autres constituants dans l'eau d'autre part. On mélange les deux solutions obtenues et on homogénéise le tout au moyen d'ultrasons.

Cette lotion permet de calmer les irritations dues au rasage, appelées couramment "feu du rasage".

### Exemple 7

### Lotion anti-allergique préventive ou curative.

### Composition :

| | |
|---|---|
| Dispersion du phosphate d'un mélange de tocophérols extraits du soja, à 4 % en phosphate de tocophérols | 25,00 g |
| Ethanol | 10,00 g |
| Propylène glycol | 5,00 g |
| Excipient aqueux q.s.p. | 100,00 g |

La dispersion à 4 % en phosphate de tocophérols extraits du soja est préparée comme à l'exemple 1b) si ce n'est qu'elle est plus concentrée en phosphate de tocophérol.

Les constituants de la formule ci-dessus sont mélangés entre eux et homogénéisés au moyen d'ultrasons.

### Exemple 8

### Solution colloïdale anti-asthmatique

### Composition

| | |
|---|---|
| Dispersion à 4 % de phosphate de dzéta 2-tocophérol | 12,50 g |
| Excipient aqueux tamponné + conservateur q.s.p. | 100,00 g |

La dispersion de phosphate de dzéta-2-tocophérol monosodique est préparée comme à l'exemple 1. On obtient après homogénéisation aux ultrasons une solution colloïdale que l'on incorpore ensuite à l'excipient tamponné.

Cette solution peut être utilisée en pulvérisation dans les voies respiratoires supérieures, notamment pour calmer les toux asthmatiformes.

### Exemple 9

### Solution colloïdale pour les techniques de réanimation.

### Composition

| | |
|---|---|
| Dispersion à 4 % de phosphate d'epsilon-tocophérol | 7,50 g |
| Excipient aqueux tamponné + conservateur q.s.p. | 100,00 g |

Cette composition est préparée comme à l'exemple précédent.

Elle peut être utilisée pour combattre les effets toxiques des radicaux superoxydes qui se forment lors de la mise en oeuvre de techniques de réanimation avec l'oxygène. Elle est alors administrée en instillation intratrachéale, en même temps que l'administration du mélange gazeux.

### Exemple 10

### Fond de teint anti-allergique.

### Composition

| | |
|---|---|
| Phosphate de gamma-tocophérol disodique | 0,5 g |
| émulsion pigmentée pour fond de teint | 99,5 g |

On prépare cette composition en incorporant à la phase aqueuse de l'émulsion, le phosphate de tocophérol disodique préalablement dispersé dans l'eau. On procède ensuite de manière classique pour réaliser l'émulsion pigmentée.

Ce fond de teint minimise les risques de manifestations allergiques dûs à une matière première ou à une substance allergène venant au contact de la peau.

## Revendications

1. Utilisation d'un phosphate de tocophérol autre que l'alpha-tocophérol, notamment sous sa forme dl ou d, ou de l'un de ses esters, de formule générale (I) suivante : 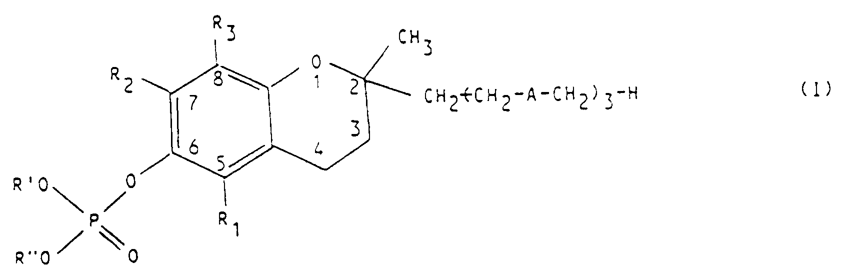 dans laquelle :
R' représente un atome d'hydrogène, un radical alkyle ayant de 1 à 4 atomes de carbone, tel qu'en particulier le radical méthyle ou éthyle, ou R'O représente un radical tocophéryle ;
R'' représente un atome d'hydrogène, un radical alkyle ayant de 1 à 4 atomes de carbone, tel qu'en particulier le radical méthyle ou éthyle, ou R''O représente une chaîne oxyéthylénée, de formule 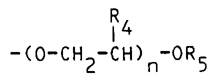 dans laquelle R₄ et R₅ représentent indépendamment un atome d'hydrogène ou un radical méthyle, et n représente un nombre entier supérieur ou égal à 1 ;
R₁, R₂ et R₃ représentent indépendamment un atome d'hydrogène ou un radical méthyle, étant entendu que R₁, R₂ et R₃ ne peuvent pas représenter simultanément un radical méthyle,
A représente les groupes : 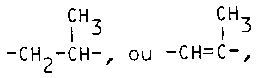 ou le dzéta 1-tocophérol ou 5,7,8-triméthyltocotriène-3',7',11'-ol, ou de l'un de ses sels, cosmétiquement ou pharmaceutiquement acceptables, pour la préparation d'une composition cosmétique ou pharmaceutique, notamment dermatologique, à potentiel allergisant ou irritant réduit, ou destiné à la prévention ou au traitement des manifestations allergiques, telles que l'allergie cutanée ou l'asthme bronchique, ou inflammatoire, ou à la prévention ou au traitement des effets nocifs des radicaux libres.

2. Utilisation selon la revendication 1, caractérisée en ce que le phosphate de tocophérol précité est le phosphate de tocol, de bêta-tocophérol ou 5,8-diméthyltocol ; gamma-tocophérol ou 7,8-diméthyltocol ; dzéta 1,2-tocophérol ou 5,7-diméthyltocol ; delta-tocophérol ou 8-méthyltocol ; êta-tocophérol ou 7-méthyltocol ; le tocotriénol, dzéta 1-tocophérol ou 5,7,8-triméthyltocotriène-3',-7',11'-ol ; epsilon-tocophérol ou 5,8-diméthyltocotriène-3',7',11'-ol ; ou gamma-tocotriénol ou delta-tocotriénol.

3. Utilisation selon la revendication 1 ou 2, caractérisée en ce qu'il s'agit d'un mélange de phosphates de tocophérol(s), dont l'un au moins est autre que le phosphate d'alpha-tocophérol, ou de leurs esters ou de leurs sels, obtenu notamment par phosphatation d'un mélange de tocophérols extrait d'une plante telle que le soja.

4. Utilisation selon l'une des revendications 1 ou 3 d'un phosphate de tocophérol, tel que précédemment défini, de préférence à l'état de sel, sous forme de petites vésicules de type liposome obtenues par dispersion dudit composé ou dudit sel dans l'eau ou dans un milieu aqueux tel qu'une solution tampon.

5. Utilisation selon la revendication 4, caractérisée en ce que la taille des vésicules est comprise environ entre 6.10⁻² µm et 2 µm.

6. Utilisation selon l'une des revendications 4 ou 5, caractérisée en ce que le milieu aqueux précité contient un agent biologiquement actif, ledit agent étant encapsulé au moins en partie après dispersion dans les vésicules précitées.

7. Utilisation selon la revendication 6, caractérisée en ce que l'agent biologiquement actif est une substance anti-allergique, telle qu'un extrait de Scutellaria comme par exemple un extrait de racine de Scutellaria Baicalensis Georgi, ou une substance anti-inflammatoire.

8. Utilisation selon l'une des revendications 1 à 7, caractérisée en ce que la concentration en poids du phosphate de tocophérol précité, ou de l'un de ses sels, ou de leurs mélanges est comprise entre 0,001 et 10 %, de préférence entre 0,01 % et 1 %, et de préférence encore entre 0,05 % et 0,5 %, par rapport au poids total de la composition.

9. Utilisation selon l'une des revendications 1 à 8, caractérisée en ce que le sel du phosphate de tocophérol précité est un sel monosodique ou le sel disodique.

10. Composition cosmétique ou pharmaceutique, notamment dermatologique, à potentiel allergisant ou irritant réduit, ou destinée notamment à la prévention ou au traitement des manifestations allergiques telles que l'allergie cutanée ou l'asthme bronchique, ou inflammatoires, ou à la prévention ou au traitement des effets nocifs des radicaux libres, caractérisée en ce qu'elle comprend une quantité cosmétiquement ou pharmaceutiquement efficace d'un phosphate de tocophérol, autre que le phosphate d'alpha-tocophérol, notamment sous sa forme dl ou d, ou de l'un de ses esters, de formule générale (I) suivante : 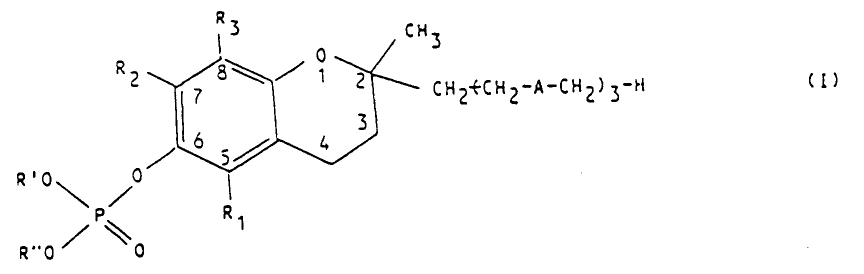 dans laquelle :
R' représente un atome d'hydrogène, un radical alkyle ayant de 1 à 4 atomes de carbone, tel qu'en particulier le radical méthyle ou éthyle, ou R'O représente un radical tocophéryle :
R'' représente un atome d'hydrogène, un radical alkyle ayant de 1 à 4 atomes de carbone, tel qu'en particulier le radical méthyle ou éthyle, ou R''O représente une chaîne oxyéthylénée, de formule 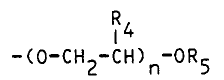 dans laquelle R₄ et R₅ représentent indépendamment un atome d'hydrogène ou un radical méthyle, et n représente un nombre entier supérieur ou égal à 1 ;
R₁, R₂ et R₃ représentent indépendamment un atome d'hydrogène ou un radical méthyle, étant entendu que R₁, R₂ et R₃ ne peuvent pas représenter simultanément un radical méthyle,
A représente les groupes : 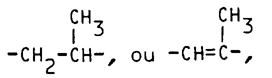 ou le dzéta 1-tocophérol ou 5,7,8-triméthyltocotriéne-3',7',11'-ol, ou de l'un de ses sels, cosmétiquement ou pharmaceutiquement acceptables.

11. Composition selon la revendication 10, caractérisée en ce que le phosphate de tocophérol précité est le phosphate de tocol, de bêta-tocophérol ou 5,8-diméthyltocol ; gama-tocophérol ou 7,8-diméthyltocol ; dzéta 1,2-tocophérol ou 5,7-diméthyltocol ; delta-tocophérol ou 8-méthyltocol ; êta-tocophérol ou 7-méthyltocol ; le tocotriénol, dzéta 1-tocophérol ou 5,7,8-triméthyltocotriène-3',7',11'-ol ; epsilon-tocophérol ou 5,8-diméthyltocotriène-3',7',11'-ol ; ou gamma-tocotriénol ou delta-tocotriénol.

12. Composition selon la revendication 10 ou 11, caractérisée en ce qu'il s'agit d'un mélange de phosphates de tocophérol(s), dont l'un au moins est autre que le phosphate d'alpha-tocophérol, ou de leurs esters ou de leurs sels, obtenus notamment par phosphatation d'un mélange de tocophérols extrait d'une plante telle que le soja.

13. Composition selon la revendication 10, 11 ou 12, caractérisée en ce qu'elle comprend une quantité cosmétiquement ou pharmaceutiquement efficace d'un phosphate de tocophérol tel que précédemment défini, de préférence à l'état de sel, sous forme de petites vésicules de type liposome obtenues par dispersion dudit composé ou dudit sel dans l'eau ou dans un milieu aqueux tel qu'une solution tampon.

14. Composition selon la revendication 13, caractérisée en ce que la taille des vésicules est comprise environ entre 6.10⁻² µm et 2 µm.

15. Composition selon l'une des revendications 10 à 14, caractérisée en ce que le milieu aqueux précité contient un agent biologiquement actif, ledit agent étant encapsulé au moins en partie après dispersion dans les vésicules précitées.

16. Composition selon la revendication 15, caractérisée en ce que l'agent biologiquement actif est une substance anti-allergique, telle qu'un extrait de Scutellaria comme par exemple un extrait de racine de Scutellaria Baicalensis Georgi, ou une substance anti-inflammatoire.

17. Composition selon l'une des revendications 10 à 16, caractérisée en ce que la concentration en poids du phosphate de tocophérol précité, ou de l'un de ses sels, est comprise entre 0,001 et 10 %, de préférence entre 0,01 % et 1 %, et de préférence encore entre 0,05 % et 0,5 %, par rapport au poids total de la composition.

18. Composition selon l'une des revendications 9 à 15, caractérisée en ce que le sel du phosphate de tocophérol précité est un sel monosodique ou le sel disodique.

19. Procédé pour diminuer le potentiel allergisant ou irritant d'une composition cosmétique ou phamaceutique, notamment dermatologique, caractérisé en ce qu'il consiste à incorporer a ladite composition une quantité cosmétiquement ou pharmaceutiquement efficace pour diminuer le potentiel allergisant ou irritant, d'au moins un phosphate de tocophérol, autre que le phosphate d'alpha-tocophérol, notamment sous sa forme dl ou d, ou de l'un de ses esters, de formule générale (I) suivante : 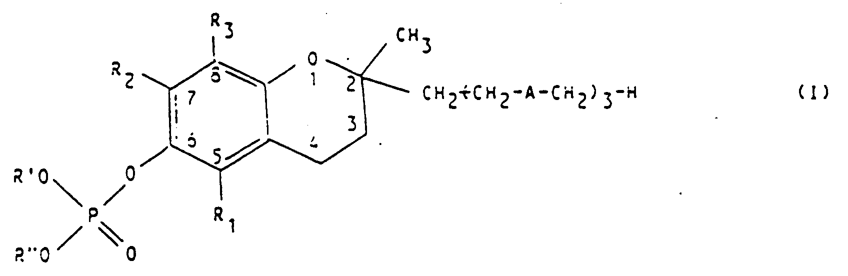 dans laquelle :
R' représente un atome d'hydrogène, un radical alkyle ayant de 1 à 4 atomes de carbone, tel qu'en particulier le radical méthyle ou éthyle, ou R'O représente un radical tocophéryle ;
R'' représente un atome d'hydrogène, un radical alkyle ayant de 1 à 4 atomes de carbone, tel qu'en particulier le radical méthyle ou éthyle, ou R''O représente une chaîne oxyéthylénée, de formule 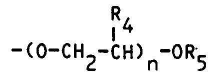 dans laquelle R₄ et R₅ représentent indépendamment un atome d'hydrogène ou un radical méthyle, et n représente un nombre entier supérieur ou égal à 1 ;
R₁, R₂ et R₃ représentent indépendamment un atome d'hydrogène ou un radical méthyle, étant entendu que R₁, R₂ et R₃ ne peuvent pas représenter simultanément un radical méthyle.
A représente les groupes : 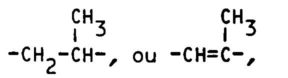 ou le dzéta 1-tocophérol ou 5,7,8-triméthyltocotriène-3',7',11'-ol, ou de l'un de ses sels, cosmétiquement ou pharmaceutiquement acceptables.

20. Procédé selon la revendication 19, caractérisé en ce que le phosphate de tocophérol précité est le phosphate de tocol, de bêta-tocophérol ou 5,8-diméthyltocol ; gamma-tocophérol ou 7,8-diméthyltocol ; dzéta 1,2-tocophérol ou 5,7-diméthyltocol ; delta-tocophérol ou 8-méthyltocol ; êta-tocophérol ou 7-méthyltocol ; tocotriénol, dzéta 1-tocophérol ou 5,7,8-triméthyltocotriène-3',7',11'-ol ; epsilon-tocophérol ou 5,8-diméthyltocotriène-3',7',-11'-ol ; ou gamma-tocotriénol ou delta-tocotriénol.

21. Procédé selon la revendication 11 ou 20, caractérisé en ce que le sel du phosphate de tocophérol précité est un sel monosodique ou le sel disodique.

22. Procédé selon l'une des revendications 19 à 21, caractérisé en ce que la concentration en poids du phosphate de tocophérol précité, ou de l'un de ses sels, est comprise entre 0,001 et 10 %, de préférence entre 0,01 % et 1 %, et de préférence encore entre 0,05 % et 0,5 %, par rapport au poids total de la composition.

23. Phosphate de tocophérol, caractérisé en ce qu'il est choisi parmi le groupe consistant du monophosphate ou diphosphate de tocol, de tocotriénol, d'epsilon-tocophérol, de dzéta 1-tocophérol, de dzéta 2-tocophérol, d'êta-tocophérol, le diphosphate de bêta-tocophérol, le diphosphate de gamma-tocophérol et le diphosphate de delta-tocophérol, ainsi que ses sels et esters.

## Claims

1. Use of a tocopherol phosphate other than alpha-tocopherol phosphate, especially in its dl or d form, or of an ester thereof, of general formula (I) below: 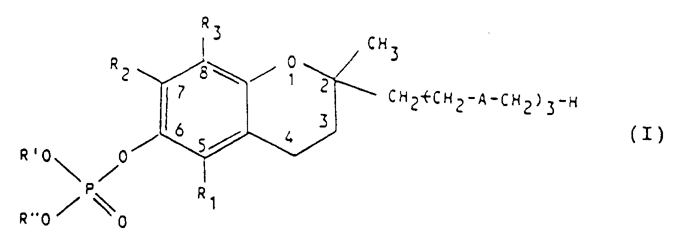 in which:
R' is a hydrogen atom or an alkyl radical having from 1 to 4 carbon atoms, such as the methyl or ethyl radical in particular, or R'O is a tocopheryl radical;
R'' is a hydrogen atom or an alkyl radical having from 1 to 4 carbon atoms, such as the methyl or ethyl radical in particular, or R''O is an oxyethylenated chain of the formula 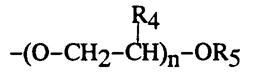 in which R₄ and R₅ independently are a hydrogen atom or a methyl radical and n is an integer greater than or equal to 1;
R₁, R₂ and R₃ independently are a hydrogen atom or a methyl radical, it being understood that R₁, R₂ and R₃ cannot simultaneously be a methyl radical,
A is the groups : 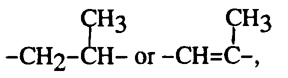 or dzeta-1-tocopherol or 5,7,8-trimethyltocotrien-3',7',11'-ol, or of a cosmetically or pharmaceutically acceptable salt thereof, for the preparation of a cosmetic or pharmaceutical composition, especially dermatological composition, having a reduced allergizing or irritating potential or intended for the prevention or treatment of allergic manifestations such as skin allergy or bronchial asthma, or inflammatory manifestations, or for the prevention or treatment of the harmful effects of free radicals.

2. Use according to claim 1, characterised in that the above-mentioned tocopherol phosphate is the phosphate of tocol, beta-tocopherol or 5,8-dimethyltocol; gamma-tocopherol or 7,8-dimethyltocol; dzeta-1,2-tocopherol or 5,7-dimethyltocol; delta-tocopherol or 8-methyltocol; eta-tocopherol or 7-methyltocol; tocotrienol, dzeta-1-tocopherol or 5,7,8-trimethyltocotrien-3',7',11'-ol; epsilon-tocopherol or 5,8-dimethyltocotrien-3',7',11'-ol; gamma-tocotrienol or delta-tocotrienol.

3. Use according to claim 1 or 2, characterised in that it is a mixture of tocopherol phosphates, at least one of which is other than alpha-tocopherol phosphate, or of esters or salts thereof, obtained especially by the phosphatization of a mixture of tocopherols extracted from a plant such as soya.

4. Use according to one of claims 1 or 3 of a tocopherol phosphate as defined above, preferably as a salt, in the form of small liposome-type vesicles obtained by the dispersion of said compound or said salt in water or an aqueous medium such as a buffer solution.

5. Use according to claim 4, characterised in that the size of the vesicles is between about 6.10⁻² µm and 2 µm.

6. Use according to one of claims 4 or 5, characterised in that the above-mentioned aqueous medium contains a biologically active agent, said agent being at least partially encapsulated, after dispersion, in the above-mentioned vesicles.

7. Use according to claim 6, characterised in that the biologically active agent is an anti-allergic substance such as an extract of Scutellaria, for example a root extract of Scutellaria baicalensis Georgi, or an anti-inflammatory substance.

8. Use according to one of claims 1 to 7, characterised in that the concentration by weight of the above-mentioned tocopherol phosphate, of a salt thereof or of mixtures thereof is between 0.001 and 10%, preferably between 0.01% and 1% and particularly preferably between 0.05% and 0.5%, with respect to the total weight of the composition.

9. Use according to one of claims 1 to 8, characterised in that the salt of the above-mentioned tocopherol phosphate is a monosodium salt or the disodium salt.

10. A cosmetic or pharmaceutical composition, especially dermatological composition, having a reduced allergizing or irritating potential or intended especially for the prevention or treatment of allergic manifestations such as skin allergy or bronchial asthma, or inflammatory manifestations, or for the prevention or treatment of the harmful effects of free radicals, said composition being characterised in that it comprises a cosmetically or pharmaceutically effective amount of a tocopherol phosphate other than alpha-tocopherol phosphate, especially in its dl or d form, or of an ester thereof, of general formula (I) below: 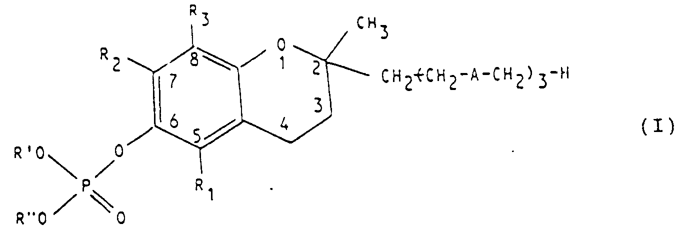 in which:
R' is a hydrogen atom or an alkyl radical having from 1 to 4 carbon atoms, such as the methyl or ethyl radical in particular, or R'O is a tocopheryl radical;
R'' is a hydrogen atom or an alkyl radical having from 1 to 4 carbon atoms, such as the methyl or ethyl radical in particular, or R''O is an oxyethylenated chain of the formula 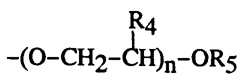 in which R₄ and R₅ independently are a hydrogen atom or a methyl radical and n is an integer greater than or equal to 1;
R₁, R₂ and R₃ independently are a hydrogen atom or a methyl radical, it being understood that R₁, R₂ and R₃ cannot simultaneously be a methyl radical.
A is the groups : 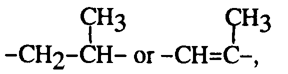 or dzeta-1-tocopherol or 5,7,8-trimethyltocotrien-3',7',11'-ol, or of a cosmetically or pharmaceutically acceptable salt thereof.

11. A composition according to claim 10, characterised in that the above-mentioned tocopherol phosphate is the phosphate of tocol, beta-tocopherol or 5,8-dimethyltocol; gamma-tocopherol or 7,8-dimethyltocol; dzeta-1,2-tocopherol or 5,7-dimethyltocol; delta-tocopherol or 8-methyltocol; eta-tocopherol or 7-methyltocol; tocotrienol, dzeta-1-tocopherol or 5,7,8-trimethyltocotrien-3',7',11'-ol; epsilon-tocopherol or 5,8-dimethyltocotrien-3',7',11'-ol; gamma-tocotrienol or delta-tocotrienol.

12. A composition according to claim 10 or 11, characterised in that it is a mixture of tocopherol phosphates, at least one of which is other than alpha-tocopherol phosphate, or of esters or salts thereof, obtained especially by the phosphatization of a mixture of tocopherols extracted from a plant such as soya.

13. A composition according to claim 10, 11 or 12, characterised in that it comprises a cosmetically or pharmaceutically effective amount of a tocopherol phosphate as defined above, preferably as a salt, in the form of small liposome-type vesicles obtained by the dispersion of said compound or said salt in water or an aqueous medium such as a buffer solution.

14. A composition according to claim 13, characterised in that the size of the vesicles is between about 6.10⁻² µm and 2 µm.

15. A composition according to one of claims 10 to 14, characterised in that the above-mentioned aqueous medium contains a biologically active agent, said agent being at least partially encapsulated, after dispersion, in the above-mentioned vesicles.

16. A composition according to claim 15, characterised in that the biologically active agent is an anti-allergic substance such as an extract of Scutellaria, for example a root extract of Scutellaria baicalensis Georgi, or an anti-inflammatory substance.

17. A composition according to one of claims 10 to 16, characterised in that the concentration by weight of the above-mentioned tocopherol phosphate or a salt thereof is between 0.001 and 10%, preferably between 0.01% and 1% and particularly preferably between 0.05% and 0.5%, with respect to the total weight of the composition.

18. A composition according to one of claims 9 to 15, characterised in that the salt of the above-mentioned tocopherol phosphate is a monosodium salt or the disodium salt.

19. A process for reducing the allergizing or irritating potential of a cosmetic or pharmaceutical composition, especially dermatological composition, characterised in that it consists in incorporating into said composition an amount, which is cosmetically or pharmaceutically effective for reducing the allergizing or irritating potential, of at least one tocopherol phosphate other than alpha-tocopherol phosphate, especially in its dl or d form, or of an ester thereof, of general formula (I) below: 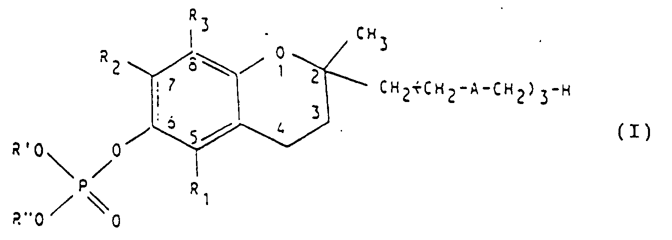 in which:
R' is a hydrogen atom or an alkyl radical having from 1 to 4 carbon atoms, such as the methyl or ethyl radical in particular, or R'O is a tocopheryl radical;
R'' is a hydrogen atom or an alkyl radical having from 1 to 4 carbon atoms, such as the methyl or ethyl radical in particular, or R''O is an oxyethylenated chain of the formula 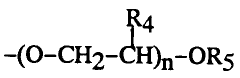 in which R₄ and R₅ independently are a hydrogen atom or a methyl radical and n is an integer greater than or equal to 1;
R₁, R₂ and R₃ independently are a hydrogen atom or a methyl radical, it being understood that R₁, R₂ and R₃ cannot simultaneously be a methyl radical.
A is the groups : 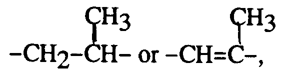 or dzeta-1-tocopherol or 5,7,8-trimethyltocotrien-3',7',11'-ol, or of a cosmetically or pharmaceutically acceptable salt thereof.

20. A process according to claim 19, characterised in that the above-mentioned tocopherol phosphate is the phosphate of tocol, beta-tocopherol or 5,8-dimethyltocol; gamma-tocopherol or 7,8-dimethyltocol; dzeta-1,2-tocopherol or 5,7-dimethyltocol; delta-tocopherol or 8-methyltocol; eta-tocopherol or 7-methyltocol; tocotrienol, dzeta-1-tocopherol or 5,7,8-trimethyltocotrien-3',7',11'-ol; epsilon-tocopherol or 5,8-dimethyltocotrien-3',7',11'-ol; gamma-tocotrienol or delta-tocotrienol.

21. A process according to claim 11 or 20, characterised in that the salt of the above-mentioned tocopherol phosphate is a monosodium salt or the disodium salt.

22. A process according to one of claims 19 to 21, characterised in that the concentration by weight of the above-mentioned tocopherol phosphate or a salt thereof is between 0.001 and 10%, preferably between 0.01% and 1% and particularly preferably between 0.05% and 0.5%, with respect to the total weight of the composition.

23. A tocopherol phosphate, characterised in that it is selected from the group consisting of tocol, tocotrienol, epsilon-tocopherol, dzeta-1-tocopherol, dzeta-2-tocepherol or eta-tocopherol monophosphate or diphosphate, beta-tocopherol diphosphate, gamma-tocopherol diphosphate and delta-tocopherol diphosphate, as well as salts and esters thereof.

## Patentansprüche

1. Verwendung eines Phosphats von Tocopherol, das von α-Tocopherol verschieden ist, insbesondere in seiner dl- oder d-Form, oder eines seiner Ester, der folgenden allgemeinen Formel (I): 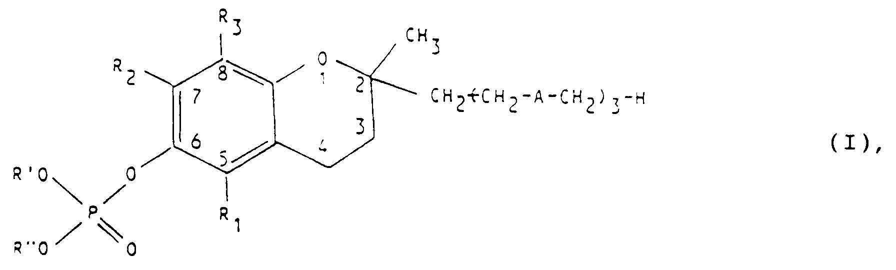 worin
R' ein Wasserstoffatom, einen Alkylrest mit 1 bis 4 Kohlenstoffatomen, wie insbesondere einen Methyl- oder Ethylrest, bedeutet, oder R'O einen Tocopherylrest darstellt;
R'' ein Wasserstoffatom, einen Alkylrest mit 1 bis 4 Kohlenstoffatomen, wie insbesondere einen Methyl- oder Ethylrest, bedeutet, oder R''O darstellt: eine oxyethylenierte Kette der Formel 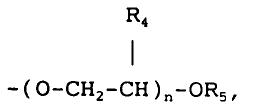 wobei R₄ und R₅ unabhängig ein Wasserstoffatom oder einen Methylrest bedeuten, und n eine ganze Zahl größer oder gleich 1 ist;
R₁, R₂ und R₃ unabhängig ein Wasserstoffatom oder einen Methylrest darstellen, mit der Maßgabe, daß R₁, R₂ und R₃ nicht gleichzeitig einen Methylrest bedeuten können;
A die Gruppen darstellt: 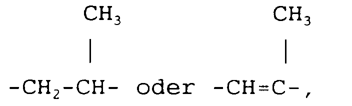 oder von ζ-1-Tocopherol oder 5,7,8-Trimethyltocotrien-3',7',11'-ol, oder eines seiner kosmetisch oder pharmazeutisch annehmbaren Salze, bei der Herstellung einer kosmetischen oder pharmazeutischen, insbesondere dermatologischen Zusammensetzung mit reduziertem Allergie- oder Reizpotential, oder zur Prävention oder Behandlung von allergischen Manifestationen, wie Hautallergien oder Asthma bronchiale, oder entzündlichen Manifestationen, oder zur Prävention oder zur Behandlung schädlicher Wirkungen freier Radikale.

2. Verwendung nach Anspruch 1, dadurch gekennzeichnet, daß das Phosphat von Tocopherol ist: das Phosphat von Tocol, β-Tocopherol oder 5,8-Dimethyltocol; γ-Tocopherol oder 7,8-Dimethyltocol; ζ-1,2-Tocopherol oder 5,7-Dimethyltocol; δ-Tocopherol oder 8-Methyltocol; η-Tocopherol oder 7-Methyltocol; Tocotrienol, ζ-1-Tocopherol oder 5,7,8,-Trimethyltocotrien-3',7',11'-ol; ε-Tocopherol oder 5,8-Dimethyltocotrien-3',7',11'-ol; oder γ-Tocotrienol oder δ-Tocotrienol.

3. Verwendung nach Anspruch 1 oder 2, dadurch gekennzeichnet, daß es sich um eine Mischung von Phosphaten von Tocopherol(en), von denen zumindest eines vom Phosphat von α-Tocopherol verschieden ist, oder ihrer Ester oder ihrer Salze handelt, die insbesondere durch Phosphatierung einer Mischung von aus einer Pflanze, wie Soja, extrahierten Tocopherolen erhalten wird.

4. Verwendung, nach einem der Ansprüche 1 oder 3, eines Tocopherolphosphats, wie vorstehend definiert, vorzugsweise im Zustand eines Salzes, in Form kleiner Vesikel vom Liposomen-Typ, die durch Dispersion der Verbindung oder des Salzes in Wasser oder in einem wässerigen Medium, wie einer Puffer-Lösung, erhalten werden.

5. Verwendung nach Anspruch 4, dadurch gekennzeichnet, daß die Größe der Vesikel zwischen etwa 6 x 10⁻² µm und 2 µm liegt.

6. Verwendung nach einem der Ansprüche 4 oder 5, dadurch gekennzeichnet, daß das wässerige Medium ein biologisch aktives Mittel enthält, welches Mittel zumindest teilweise nach der Dispersion in den Vesikeln eingekapselt wird.

7. Verwendung nach Anspruch 6, dadurch gekennzeichnet, daß das biologisch aktive Mittel eine anti-allergische Substanz, wie ein Scutellaria-Extrakt, wie beispielsweise ein Wurzelextrakt von Scutellaria baicalensis georgi, oder eine entzündungshemmende Substanz ist.

8. Verwendung nach einem der Ansprüche 1 bis 7, dadurch gekennzeichnet, daß die Massekonzentration des Tocopherolphosphats, oder eines seiner Salze, oder ihrer Mischungen, zwischen 0,001 und 10 %, vorzugsweise zwischen 0,01 % und 1 % und bevorzugter zwischen 0,05 % und 0,5 %, bezogen auf die Gesamtmasse der Zusammensetzung, beträgt.

9. Verwendung nach einem der Ansprüche 1 bis 8, dadurch gekennzeichnet, daß das Salz des Tocopherolphosphats ein Mononatriumsalz oder ein Dinatriumsalz ist.

10. Kosmetische oder pharmazeutische, insbesondere dermatologische Zusammensetzung mit reduziertem Allergie- oder Reizpotential, oder insbesondere zur Prävention oder Behandlung von allergischen Manifestationen, wie Hautallergien oder Asthma bronchiale, oder entzündlichen Manifestationen, oder zur Prävention oder zur Behandlung schädlicher Wirkungen freier Radikale, dadurch gekennzeichnet, daß sie umfaßt: eine kosmetisch oder pharmazeutisch wirksame Menge eines Phosphats von Tocopherol, das von α-Tocopherol verschieden ist, insbesondere in seiner dl- oder d-Form, oder eines seiner Ester, der folgenden allgemeinen Formel (I): 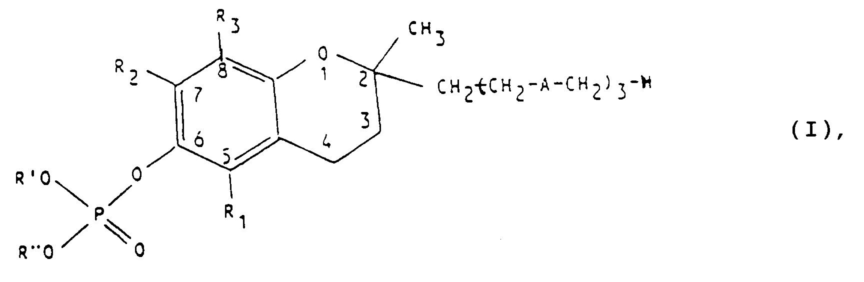 worin
R' ein Wasserstoffatom, einen Alkylrest mit 1 bis 4 Kohlenstoffatomen, wie insbesondere einen Methyl- oder Ethylrest, bedeutet, oder R'O einen Tocopherylrest darstellt;
R'' ein Wasserstoffatom, einen Alkylrest mit 1 bis 4 Kohlenstoffatomen, wie insbesondere einen Methyl- oder Ethylrest, bedeutet, oder R''O darstellt: eine oxyethylenierte Kette der Formel 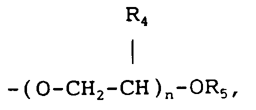 wobei R₄ und R₅ unabhängig ein Wasserstoffatom oder einen Methylrest bedeuten, und n eine ganze Zahl größer oder gleich 1 ist;
R₁, R₂ und R₃ unabhängig ein Wasserstoffatom oder einen Methylrest darstellen, mit der Maßgabe, daß R₁, R₂ und R₃ nicht gleichzeitig einen Methylrest bedeuten können;
A die Gruppen darstellt: 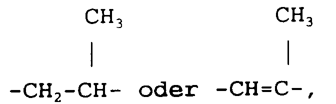 oder von ζ-1-Tocopherol oder 5,7,8-Trimethyltocotrien-3',7',11'-ol, oder eines seiner kosmetisch oder pharmazeutisch annehmbaren Salze.

11. Zusammensetzung nach Anspruch 10, dadurch gekennzeichnet, daß das Phosphat von Tocopherol ist: das Phosphat von Tocol, β-Tocopherol oder 5,8-Dimethyltocol; γ-Tocopherol oder 7,8-Dimethyltocol; ζ-1,2-Tocopherol oder 5,7-Dimethyltocol; δ-Tocopherol oder 8-Methyltocol; η-Tocopherol oder 7-Methyltocol; Tocotrienol, ζ-1-Tocopherol oder 5,7,8-Trimethyltocotrien-3',7',11'-ol; ε-Tocopherol oder 5,8-Dimethyltocotrien-3',7',11'-ol; oder γ-Tocotrienol oder δ-Tocotrienol.

12. Zusammensetzung nach Anspruch 10 oder 11, dadurch gekennzeichnet, daß es sich um eine Mischung von Phosphaten von Tocopherol(en), von denen zumindest eines vom Phosphat von α-Tocopherol verschieden ist, oder ihrer Ester oder ihrer Salze handelt, die insbesondere durch Phosphatierung einer Mischung von aus einer Pflanze, wie Soja, extrahierten Tocopherolen erhalten wird.

13. Zusammensetzung nach Anspruch 10, 11 oder 12, dadurch gekennzeichnet, daß sie eine kosmetisch oder pharmazeutisch wirksame Menge eines Tocopherolphosphats, wie vorstehend definiert, vorzugsweise im Zustand eines Salzes, in Form kleiner Vesikel vom Liposomen-Typ umfaßt, die durch Dispersion der Verbindung oder des Salzes in Wasser oder in einem wässerigen Medium, wie einer Puffer-Lösung, erhalten werden.

14. Zusammensetzung nach Anspruch 13, dadurch gekennzeichnet, daß die Größe der Vesikel zwischen etwa 6 x 10⁻² µm und 2 µm liegt.

15. Zusammensetzung nach einem der Ansprüche 10 bis 14, dadurch gekennzeichnet, daß das wässerige Medium ein biologisch aktives Mittel enthält, welches Mittel zumindest teilweise nach der Dispersion in den Vesikeln eingekapselt wird.

16. Zusammensetzung nach Anspruch 15, dadurch gekennzeichnet, daß das biologisch aktive Mittel eine anti-allergische Substanz, wie ein Scutellaria-Extrakt, wie beispielsweise ein Wurzelextrakt von Scutellaria baicalensis georgi, oder eine entzündungshemmende Substanz ist.

17. Zusammensetzung nach einem der Ansprüche 10 bis 16, dadurch gekennzeichnet, daß die Massekonzentration des Tocopherolphosphats, oder eines seiner Salze, zwischen 0,001 und 10 %, vorzugsweise zwischen 0,01 % und 1 % und bevorzugter zwischen 0,05 % und 0,5 %, bezogen auf die Gesamtmasse der Zusammensetzung, beträgt.

18. Zusammensetzung nach einem der Ansprüche 9 bis 15, dadurch gekennzeichnet, daß das Salz des Tocopherolphosphats ein Mononatriumsalz oder ein Dinatriumsalz ist.

19. Verfahren zur Verringerung des Allergie- oder Reizpotentials einer kosmetischen oder pharmazeutischen, insbesondere dermatologischen Zusammensetzung, dadurch gekennzeichnet, daß es darin besteht, daß in die Zusammensetzung eingeschlossen wird: eine kosmetisch oder pharmazeutisch zur Verringerung des Allergie- oder Reizpotentials wirksame Menge zumindest eines Tocopherolphosphats, das von α-Tocopherolphosphat verschieden ist, insbesondere in seiner dl- oder d-Form, oder eines seiner Ester, der folgenden allgemeinen Formel (I): 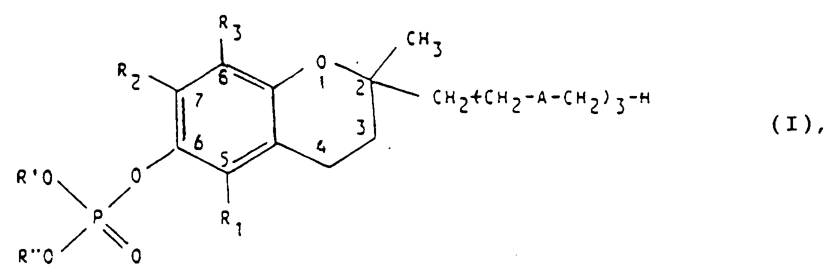 worin
R' ein Wasserstoffatom, einen Alkylrest mit 1 bis 4 Kohlenstoffatomen, wie insbesondere einen Methyl- oder Ethylrest, bedeutet, oder R'O einen Tocopherylrest darstellt;
R'' ein Wasserstoffatom, einen Alkylrest mit 1 bis 4 Kohlenstoffatomen, wie insbesondere einen Methyl- oder Ethylrest, bedeutet, oder R''O darstellt: eine oxyethylenierte Kette der Formel 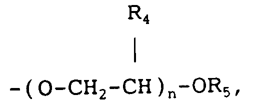 wobei R₄ und R₅ unabhängig ein Wasserstoffatom oder einen Methylrest bedeuten, und n eine ganze Zahl größer oder gleich 1 ist;
R₁, R₂ und R₃ unabhängig ein Wasserstoffatom oder einen Methylrest darstellen, mit der Maßgabe, daß R₁, R₂ und R₃ nicht gleichzeitig einen Methylrest bedeuten können;
A die Gruppen darstellt: 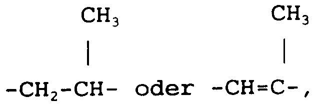 oder von ζ-1-Tocopherol oder 5,7,8-Trimethyltocotrien-3',7',11'-ol, oder eines seiner kosmetisch oder pharmazeutisch annehmbaren Salze.

20. Verfahren nach Anspruch 19, dadurch gekennzeichnet, daß das Phosphat von Tocopherol ist: das Phosphat von Tocol, β-Tocopherol oder 5,8-Dimethyltocol; γ-Tocopherol oder 7,8-Dimethyltocol; ζ-1,2-Tocopherol oder 5,7-Dimethyltocol; δ-Tocopherol oder 8-Methyltocol; η-Tocopherol oder 7-Methyltocol; Tocotrienol, ζ-1-Tocopherol oder 5,7,8-Trimethyltocotrien-3',7',11'-ol; ε-Tocopherol oder 5,8-Dimethyltocotrien-3',7',11'-ol; oder γ-Tocotrienol oder δ-Tocotrienol.

21. Verfahren nach Anspruch 11 oder 20, dadurch gekennzeichnet, daß das Salz des Tocopherolphosphats ein Mononatriumsalz oder ein Dinatriumsalz ist.

22. Verfahren nach einem der Ansprüche 19 bis 21, dadurch gekennzeichnet, daß die Massekonzentration des Tocopherolphosphats, oder eines seiner Salze, zwischen 0,001 und 10 %, vorzugsweise zwischen 0,01 % und 1 % und bevorzugter zwischen 0,05 % und 0,5 %, bezogen auf die Gesamtmasse der Zusammensetzung, beträgt.

23. Tocopherolphosphat, dadurch gekennzeichnet, daß es ausgewählt ist aus der Gruppe bestehend aus Monophosphat oder Diphosphat von Tocol, Tocotrienol, ε-Tocopherol, ζ-1-Tocopherol, ζ-2-Tocopherol, η-Tocopherol, β-Tocopheroldiphosphat, γ-Tocopheroldiphosphat und δ-Tocopheroldiphosphat, sowie ihren Salzen und Estern.
